# EUROPEAN PATENT APPLICATION

(11) **EP 4 176 798 A1**
(43) Date of publication of application: **10.05.2023**
(21) Application number: 22192296.6
(22) Date of filing: 26.08.2022
(51) Int. Cl.: A61B 5/00, A61B 5/11, G06T 13/00, G06T 15/00, G06T 13/40, A63B 71/00, G06T 7/292

(54) **MONITORING EXERCISE ACTIVITY IN A GYM ENVIRONMENT**

(30) Priority: 05.11.2021 SE 2151357
(71) Applicant: Sony Group Corporation, Minato-Ku, Tokyo, 108-0075 (JP)
(72) Inventor: HÅKANSSON, Jakob, Basingstoke, RG22 4SB (GB); MULDER, Axel, Basingstoke, RG22 4SB (GB)
(74) Representative: Neij & Lindberg AB

(57) **Abstract**

A system for monitoring exercise activity in a gym environment comprises an image-based monitoring arrangement (10), a storage device (31), and a replay device (30, 30'). The monitoring arrangement (10) generates a time series of 3D representations of an individual, with each 3D representation defining a pose of the individual and comprising 3D positions of a plurality of predefined feature points of the individual. The storage device (31) receives and stores an exercise sequence, which comprises at least part of the time series of 3D representations and corresponds to an exercise performed by the individual. The replay device (30; 30') retrieves the exercise sequence from the storage device (31), renders an animation of the individual when performing the exercise based on the exercise sequence, and provides the animation for display. The system enables a user to review the exercise at any desirable viewing angle and/or magnification.

## Description

### Technical Field

The present disclosure relates generally to techniques for monitoring activity in a gym environment and, in particular, to improving the performing of exercise activities by individuals in a gym environment.

### Background Art

In recent years, there has been a virtual explosion in the popularity of exercise and physical fitness. There are many popular forms of physical exercise including, for example, running, bicycling, and strength training. The growing interest in strength training is reflected by the growing number of gyms found in both public and private settings. Gyms may offer possibilities for weight training in machines or by use of free weights, as well as body weight training and group training in gym classes.

When exercising, it is crucial to perform each exercise with a correct technique or "form" to reduce the risk of injury, prevent cheating and improve performance in terms of muscle gain, strength, explosiveness, etc. An individual may practice good form in front of a mirror or by following advice from a so-called spotter, which may be a hired trainer or an accompanying friend. The use of a mirror is typically limited to a front view of the exercise, and the use of a trainer or friend may not be possible at times. A hired trainer is also expensive. Further, the accompanying friend may lack sufficient experience to provide adequate feedback on how the exercise is performed. As handheld devices with imaging capability, such as digital cameras and mobile phones, are commonplace, it is also possible for the individual to arrange such a handheld device to capture video footage of the exercise and then review the result afterwards. Here, the viewing angle of the exercise is again limited. Further, it may be difficult to properly arrange the handheld device, and the individual may lack the experience to be able to identify bad form.

### Brief Summary

It is an objective to at least partly overcome one or more limitations of the prior art.

Another objective is to provide a technique of facilitating review of an exercise performed by an individual in a gym environment.

Yet another objective is to provide such a technique that enables the review to be performed from plural viewing angles onto the individual.

One or more of these objectives, as well as further objectives that may appear from the description below, are at least partly achieved by a system for monitoring exercise activity in a gym environment, a replay device for use in the system, and a method of monitoring exercise activity according to the independent claims, embodiments thereof being defined by the dependent claims.

Still other objectives, as well as features, aspects and technical effects will appear from the following detailed description, from the attached claims as well as from the drawings.

### Brief Description of Drawings

FIG. 1 is a top plan view of an individual performing an exercise in a gym environment comprising an image-based monitoring system.
FIGS 2A-2B schematically depict a 2D image and a 3D representation, respectively, of an individual performing an exercise.
FIG. 3 is a block diagram of an example system for monitoring exercise activity in a gym environment.
FIG. 4 is a flow chart of an example method of monitoring exercise activity in a gym environment.
FIGS 5A-5B show, from two different viewing angles, an animated image of an individual performing an exercise.
FIG. 6 is a flow chart of an example procedure that may be part of the method in FIG. 3.
FIG. 7 is a block diagram of a sub-system for detecting and evaluating deviations in the performance of an exercise.
FIG. 8 shows an animated image with corrective feedback.
FIG. 9 is a block diagram of an example machine that may implement one or more functions depicted in FIGS 4 and 6.

### Detailed Description of Example Embodiments

Embodiments will now be described more fully hereinafter with reference to the accompanying schematic drawings, in which some, but not all, embodiments are shown. Indeed, the subject of the present disclosure may be embodied in many different forms and should not be construed as limited to the embodiments set forth herein; rather, these embodiments are provided so that this disclosure may satisfy applicable legal requirements.

Also, it will be understood that, where possible, any of the advantages, features, functions, devices, and/or operational aspects of any of the embodiments described and/or contemplated herein may be included in any of the other embodiments described and/or contemplated herein, and/or vice versa. In addition, where possible, any terms expressed in the singular form herein are meant to also include the plural form and/or vice versa, unless explicitly stated otherwise. As used herein, "at least one" shall mean "one or more" and these phrases are intended to be interchangeable. Accordingly, the terms "a" and/or "an" shall mean "at least one" or "one or more", even though the phrase "one or more" or "at least one" is also used herein. As used herein, except where the context requires otherwise owing to express language or necessary implication, the word "comprise" or variations such as "comprises" or "comprising" is used in an inclusive sense, that is, to specify the presence of the stated features but not to preclude the presence or addition of further features in various embodiments.

As used herein, the terms "multiple", "plural" and "plurality" are intended to imply provision of two or more elements, whereas the term a "set" of elements is intended to imply a provision of one or more elements. The term "and/or" includes any and all combinations of one or more of the associated listed elements.

It will furthermore be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing the scope of the present disclosure.

Well-known functions or constructions may not be described in detail for brevity and/or clarity. Unless otherwise defined, all terms (including technical and scientific terms) used herein have the same meaning as commonly understood by one of ordinary skill in the art to which this disclosure belongs.

Like numerals refer to like elements throughout.

Before describing embodiments in more detail, a few definitions will be given.

As used herein, "keypoint" has its conventional meaning in the field of computer vision and is also known as an interest point. A keypoint is a spatial location or point in an image that define what is interesting or what stand out in the image and may be defined to be invariant to image rotation, shrinkage, translation, distortion, etc. More generally, a keypoint may be denoted a "reference point" or "feature point" on an object to be detected in the image, with the reference point having a predefined placement on the object. For humans, keypoints may identify joints and/or extremities. Keypoints may be detected by use of any existing feature detection algorithm(s), for example image processing techniques that are operable to detect one or more of edges, corners, blobs, ridges, etc. in digital images. Non-limiting examples of feature detection algorithms comprise SIFT (Scale-Invariant Feature Transform), SURF (Speeded Up Robust Feature), FAST (Features from Accelerated Segment Test), SUSAN (Smallest Univalue Segment Assimilating Nucleus), Harris affine region detector, and ORB (Oriented FAST and Rotated BRIEF). Further information about conventional keypoint detectors is found in the article "Local invariant feature detectors: a survey", by Tuytelaars et al, published in Found. Trends. Comput. Graph. Vis. 3(3), 177-280 (2007). Further examples of feature detection algorithms are found in the articles "Simple Baselines for Human Pose Estimation and Tracking", by Xiao et al, published at ECCV 2018, and "Deep High-Resolution Representation Learning for Human Pose Estimation", by Sun et al, published at CVPR 2019. Correspondingly, objects may be detected in images by use of any existing object detection algorithm(s). Non-limiting examples include various machine learning-based approaches or deep learning-based approaches, such as Viola-Jones object detection framework, SIFT, HOG (Histogram of Oriented Gradients), Region Proposals (RCNN, Fast-RCNN, Faster-RCNN), SSD (Single Shot MultiBox Detector), You Only Look Once (YOLO, YOLO9000, YOLOv3), and RefineDet (Single-Shot Refinement Neural Network for Object Detection).

As used herein, "pose" defines the posture of a human object and comprises a collection of positions which may represent keypoints. The positions may be two-dimensional (2D) positions, for example in an image coordinate system, resulting in a 2D pose, or three-dimensional (3D) positions, for example in a scene coordinate system, resulting in a 3D pose. A pose for a human object is also referred to as a "skeleton" herein.

As used herein, "gym environment" refers to a place, indoors or outdoors, at which individuals perform physical activities by use of machines, free weights, body weight or other equipment, individually or in groups, for the purpose of exercise or training.

As used herein, "image-based monitoring system" refers to a system that comprises one or more imaging devices and is configured to generate a time sequence of 3D poses of an individual based on images taken by the one or more imaging devices. The imaging device(s) may be responsive to radiation in any wavelength range, for example visible radiation and/or IR radiation. The system may be configured to generate the respective 3D pose based on 2D images taken by two or more imaging devices arranged at different viewing angles onto the individual, or based on image(s) taken by one or more imaging devices capable of depth-sensing, for example as implemented by Microsoft Kinect^{™}, or any combination thereof.

As used herein, "animation" refers to a plurality of animated images which, when displayed in sequence, appear as moving images. The animated images may be generated by computer graphics.

As used herein, "avatar" refers to an animated representation of a human individual. The avatar may represent the skeleton and/or the skeletal muscles of the individual. The avatar may be generic and represent all or a group of individuals. Alternatively, the avatar may be customizable to mimic the visual appearance of a specific individual or group of individuals.

Embodiments relate to monitoring of physical activities performed by individuals or users in a gym environment and is based on the insight that it is possible to both enhance and simplify the review of such activities by use of 3D representations of the users. For example, a time series of 3D representations of a user may be generated and stored during training and later retrieved for presentation, by the user or someone else, at any desirable viewing angle and/or magnification. Thus, embodiments not only enable the user to review performance but also improves remote coaching, by empowering a trainer at a remote location from the gym to thoroughly review how the activity is performed by the user. A time series of 3D representations provides significantly more relevant data about an activity compared to ordinary 2D video footage. The time series of 3D representations may also be algorithmically analyzed to generate one or more objective performance indicators for presentation, for example in terms of a quantified metric or identification of deviation(s) from proper form. The performance indicator(s) may guide the individual towards better implementation of an activity, to thereby increase gains and/or reduce the risk of injury. Further, by being objectively determined, the performance indicator(s) may be useful in officiating, also outside the field of gym environments, for example as a tool for referees or umpires in sport competitions such as weightlifting, cross fit, martial arts, fencing, etc.

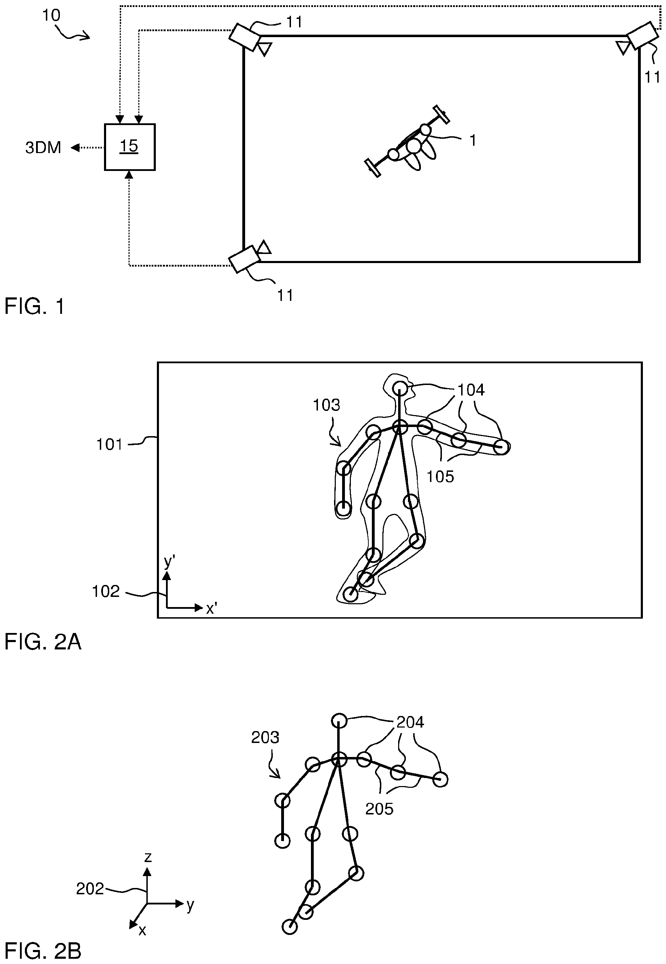

FIG. 1 is a top plan view of a gym environment, in which an individual 1 performs a physical activity, here an exercise by use of a barbell. An image-based monitoring arrangement 10 is installed in the gym environment to monitor the space where the individual 1 performs the activity. In the illustrated example, the monitoring arrangement 10 comprises a plurality of imaging devices ("cameras"), which are connected to a 3D reconstruction unit 15. The cameras 11 generate a respective stream or time series of 2D images of the individual 1, which are processed by the unit 15 into a stream or time series of 3D representations of the individual 1. The stream of 3D representations forms 3D motion data for the individual, abbreviated 3DM in the following.

FIG. 1 is merely an example. For example, any number of cameras 11 may be included in the arrangement 10. Further, the 3D reconstruction unit 15 may be incorporated in one of the cameras 11 or may be distributed among the cameras 1, assuming that the cameras 11 are connected to communicate with each other.

Any technique for 3D reconstruction and generation of the 3DM may be implemented within the context of the present disclosure. An example will be briefly described with reference to FIGS 2A-2B for the purpose of illustration only.

FIG. 2A is a 2D image 101 taken by a camera from a specific viewing angle. The image 101 includes a 2D representation 103 of an individual. The 2D representation 103 depicts a "2D pose" of the individual as seen from the viewing angle. The 2D pose is defined by predefined feature points ("keypoints") 104, which are connected by 2D links 105. The position of each keypoint is given by 2D coordinates (x',y') in a local coordinate system 102 of the image/camera. As noted above, many established techniques for detection of keypoints are available.

FIG. 2B is a 3D representation 203 of the individual in FIG. 2A. The 3D representation 203 depicts a "3D pose" of the individual. The 3D pose is defined by keypoints 204, which correspond to the keypoints 104 in FIG. 2A and are connected by links 205, which correspond to the links 105 in FIG. 2A. The position of each keypoint is given by 3D coordinates (x,y,z) in a scene coordinate system 202 in the physical space where the individual is located. The 3D pose may be generated by processing of a plurality of 2D poses of the individual from different viewing angles. Such processing is well-known in the art and may comprise identifying corresponding keypoints between the 2D poses, and triangulating the 2D coordinates of corresponding keypoints into 3D coordinates by use of calibration data representing the position and orientation of the cameras that provide the 2D poses. The resulting 3D pose is commonly known as a "skeleton" since it, depending on the selection of keypoints, may provide an outline of how different limbs of an individual is oriented in 3D space. It should be noted that the links 205 are generally implicit to the 3D pose and are shown in FIG. 2B for illustrative purposes.

The above-mentioned 3D motion data, 3DM, may be generated by processing plural streams of images taken from different viewing angles to generate corresponding streams of 2D poses, and merging the streams of 2D poses into a stream of 3D poses by triangulation.

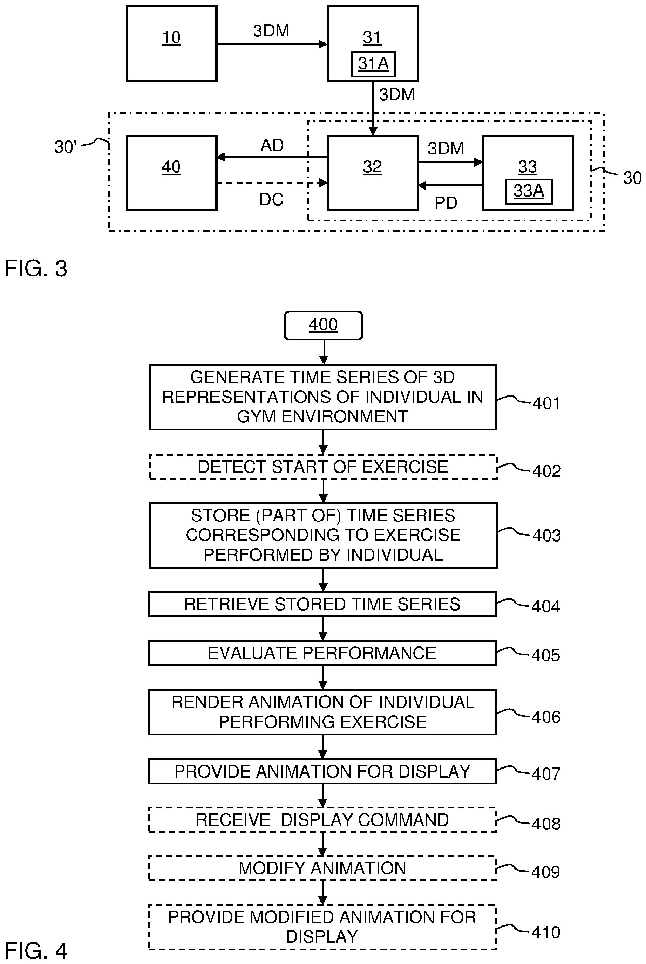

FIG. 3 is a block diagram of an example system in accordance with some embodiments. The system comprises the image-based monitoring arrangement 10, which is located at a gym and configured to provide the 3DM, which represents the motion pattern of an individual. A storage device 31 is arranged to receive the 3DM and store at least part of the 3DM in a memory unit 31A. The storage device 31 may be located at the gym, on a computer system resource in the cloud, etc. A replay device, indicated as 30 or 30' depending on implementation, is configured to selectively retrieve 3DM from the storage device 31 and render an animation of the individual when performing an exercise and provide the animation for display on a feedback device 40. As shown, the replay device 30' may comprise a rendering device 32 which is configured to render the animation based on the 3DM and provide corresponding animation data AD to the feedback device 40.

In some embodiments, as indicated by dot-dashed lines 30, the feedback device 40 is not part of the replay device. For example, the feedback device 40 may be included in a local electronic device at the location of the individual, i.e. in the gym environment, or at another location, for example at the location of a trainer. The feedback device 40 comprises a display and may also comprise an input interface, such as a keyboard, keypad, computer mouse, one or more touch buttons, voice control, touch screen, etc. The electronic device may be a smartphone, tablet computer, laptop, desktop computer, a TV, a monitor, etc. The replay device 30 is physically separated from the local electronical device and may, for example, be located on a server at the gym or on a computer system resource in the cloud.

In other embodiments, as indicated by dot-dashed lines 30', the feedback device 40 is part of the replay device, which thus may be incorporated into the above-mentioned local electronic device.

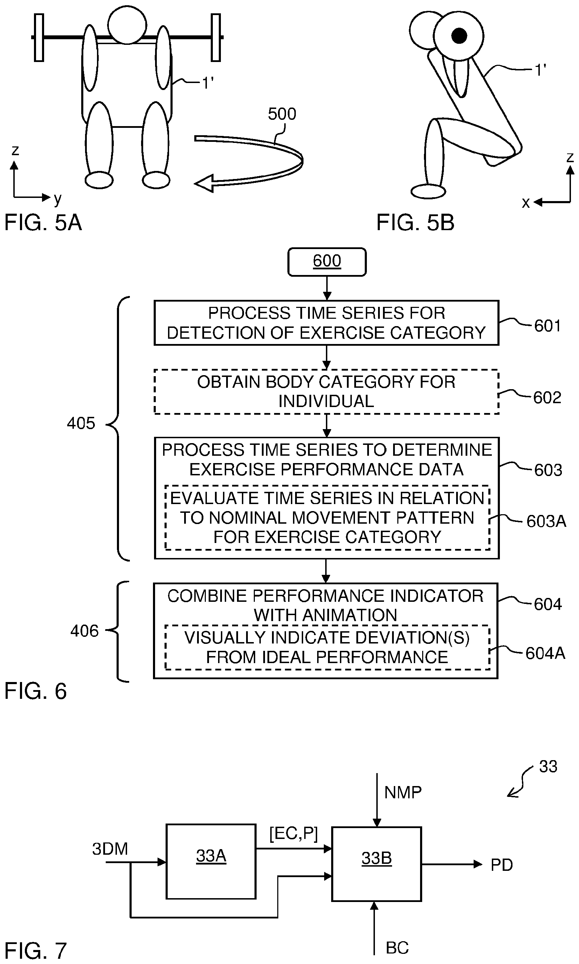

In the example of FIG. 3, the system also comprises an evaluation device 33, which may be part of the replay device 30, 30', as shown, or separate from the replay device. As will described in detail with reference to FIGS 6-8, the evaluation device 33 is configured to process the 3DM as retrieved by the replay device 30, 30' from the storage device 31, for evaluation of one or more exercises performed by the individual. The evaluation results in exercise performance data PD, which is indicative of how well the individual performs the exercise(s). The performance data PD is provided to the rendering device 32, which may include PD in the animation rendered by the rendering device 32 or separately provide PD for display on the feedback device 40.

FIG. 4 is a flowchart of an example method 400 of monitoring physical activity in a gym environment in accordance with some embodiments. The method 400 may be implemented and performed by the system in FIG. 3. Dashed boxes represent optional steps.

In step 401, the image-based monitoring arrangement 10 is operated to generate a time series of 3D representations of an individual in the gym environment. The time series corresponds to 3DM in FIGS 1 and 3. As understood from FIG. 2B, each of the 3D representations defines a 3D pose 203 of the individual and comprises positions of a plurality of keypoints 204 of the individual in a 3D coordinate system 202.

In step 403, at least part of the time series, 3DM, is stored in the storage device 31. The at least part of the 3DM corresponds to an exercise performed by the individual and is also denoted "exercise sequence" herein.

As shown, step 403 may be preceded by a step 402, in which the 3DM generated by the monitoring arrangement 10 is processed for detection of the start of an exercise. Step 402 may be performed by the monitoring arrangement 10. In one example, the arrangement 10 is configured to output the 3DM only when it has detected the start of an exercise, to thereby minimize data traffic between the arrangement 10 and the storage device 31, as well as reduce the required storage capacity of the storage device 31. In another example, the arrangement 10 provides a signal indicating the start of an exercise to the storage device 31, which is thereby caused to store the 3DM associated with the signal. Alternatively, step 403 is performed by the storage device 31. In one example, the storage device 31 is configured to only store the incoming 3DM when the start of an exercise has been detected. In another example, the storage device 31 is configured to operate at a first storage rate by default and, upon detection of the start of an exercise, operate at a second storage rate, which is higher than the first storage rate. The storage rate denotes the number of 3D poses stored per unit time. Common to all examples, irrespective of where the start of the exercise is detected, is that the storage device 31 is operable to selectively increase the storage rate, from either zero or the first storage rate, upon the detection of the start. Correspondingly, step 402 may comprise selectively decreasing the storage rate upon detection that the exercise is completed.

In step 404, the exercise sequence stored in step 403 is retrieved. In the example of FIG. 3, step 403 is performed by the replay device 30, 30'. The exercise sequence may be selected by a user via the feedback device 40, which thereby provides a display command (DC in FIG. 3) indicative of the exercise sequence to the rending device 32.

In step 406, the replay device 30, 30' renders an animation of the individual performing the exercise based on the exercise sequence. The animation is a replay of the poses attained by the individual during the exercise. In the example of FIG. 3, the rendering is performed by the rendering device 32 in the replay device 30, 30'. In some embodiments, the replay device 30, 30' is configured to render the animation as an avatar which is spatially matched to the 3D poses in the exercise sequence. An example of such an avatar 1' is shown in FIGS 5A-5B. By rendering the animation as an avatar 1', it may be easier for the viewer to compare the animated exercise to a real exercise. The avatar 1' may or may not be personalized to the individual that performs the exercise. In some embodiments, the avatar 1' is rendered to exhibit one or more physical properties of the individual that performs the exercise. The properties may be provided to the system is any suitable way. For example, they may be retrieved from a user account, extracted from 2D images captured by the monitoring arrangement 10, entered by the individual via the feedback device 40, etc. Examples of physical properties include weight, height, gender, or body category (below).

In step 407, the animation is provided for display, for example as animation data AD from the rendering device 32 to the feedback device 40 in FIG. 3. The animation may be provided so as to allow the user to select viewing angle and/or magnification ratio. In the example of FIG. 5A, a user is able to impart a selected rotation 500 to an animated individual 1' that performs squats. This allows the user to inspect the exercise performance from different viewing angles, for example from the front view in FIG. 5A and from the side view in FIG. 5B.

The animation data AD may be provided in different formats. In some embodiments, AD comprises a self-contained computer file with an interactive 360 animation, which allows a user to pan and zoom the rendered individual independent of the rendering device 32, by the user operating the computer file to select various viewing angles and/or magnifications for the replay and/or to stop the replay and zoom in on selected details. For example, such a computer file may be provided to a feedback device 40 that is physically separate from the replay device (cf. 30 in FIG. 3), to allow for off-line viewing. In some embodiments, represented by steps 408-410 in FIG. 4, the animation is controlled by the rendering device 32 during the replay on the feedback device 40. In step 408, the rendering device 32 receives from the feedback device 40 a display command that indicates a viewing angle and/or magnification ratio selected by a user, for example based on the animation provided in step 407. In step 409, the animation is modified by the rendering device 32 according to the display command. In step 410, the modified animation is provided by the rendering device 32 for display to the user. In the example of FIG. 3, AD may comprise the modified animation, which has been rendered in response to the display command, DC.

In some embodiments, the method and system described herein enable a trainer, who may be at a remote location, to review the exercise performed by the individual in the gym environment by commanding replay of the animation on a feedback device at the remote location. The replay may be performed in (near) real time with the individual's execution of the exercise, or at a separate time point. In some embodiments, the system comprises plural feedback devices, which are communicatively connected to the replay device (30 in FIG. 3). Such a system may be operable to synchronously replay the animation on two (or more) feedback devices, for example one in the gym environment and one at the remote location, thereby enabling the individual and the remote trainer to jointly review the exercise. In another example, the system is operable to transmit feedback entered on one feedback device to another feedback device in the system, via the replay device. This allows a remote trainer to provide, through the system, feedback on the individual's execution of the exercise, as represented by the animation, to the individual. The trainer's feedback may be displayed in clear text on the feedback device of the individual and may or may not be displayed together with the animation. In one implementation, the trainer is given the ability to incorporate the feedback into the animation, for example to graphically highlight certain body parts or indicate a desired range of motion, body pose, etc. In such an implementation, the system may be configured to provide the animation for display to the trainer on one feedback device; receive, at the rendering device 32, feedback data entered by the trainer on this feedback device; update, by the rendering device 32, the animation based on the feedback data; and provide the updated animation for display to the individual on another feedback device.

As shown in FIG. 4, the method 400 may comprise a step 405 for algorithmic evaluation of the individual's performance of the exercise, based on the exercise sequence retrieved in step 404. Step 405 results in the above-mentioned performance data, PD, which may comprise one or more performance indicators that are representative of the individual's performance of the exercise. The performance data PD may then, in step 406, be combined with the animation, for example included as part of the animation, and provided for display in step 407. In the system of FIG. 3, step 405 is implemented by the evaluation device 33.

FIG. 6 is flowchart of an example procedure 600 for evaluating exercise performance and providing corresponding feedback. The procedure 600 may be part of steps 405-406 in FIG. 4.

In step 601, the exercise sequence is processed for detection of an exercise category among a plurality of exercise categories. Step 601 results in a classification of the exercise sequence. The exercise category may be determined with any desired granularity or level of detail. In one example, the exercise category may indicate the type of equipment used in the exercise, such as machine, barbell, dumbbell, kettlebell, body weight, cables, etc. In another example, the exercise category may identify a general type of exercise, such as deadlift, fly, overhead press, etc. In yet another example, the exercise category may identify a specific exercise implementation, such as butterfly machine fly, inclined bench dumbbell fly, cable crossover fly, dumbbell shoulder fly, etc. Step 601 may comprise operating any available human activity recognition algorithm on the exercise sequence. In the example of FIG. 3, step 601 may be performed by a trained machine learning-based model 33A in the evaluation device 33. The model 33A may involve deep learning, for example by use of convolutional neural networks or recurrent neural networks. Examples of human activity recognition algorithms that may be adapted for use in the model 33A are found in the article "A Comprehensive Study of Deep Video Action Recognition", by Zhu et al, published in arXiv:2012.06567, December 2020, which is incorporated herein in its entirety by reference. As an alternative or supplement, the exercise category may be entered into the system by the individual, for example via the local electronic device, before or after performing the exercise, and step 602 may comprise obtaining the thus-entered exercise category.

In step 602, a body category for the individual is obtained. The body category represents the body build and/or one or more physical traits of the individual. The body category may be obtained in many different ways. For example, the body category may be prestored for the individual in a database, determined by processing images acquired by the monitoring system 10, determined by processing one or more exercise sequences, entered into the system by the individual, etc. The body category may indicate one or more of the body shape of the individual (for example, "short", "medium height", "tall", "slim", "medium weight", "heavy"), the skeletal framework of the individual, or the fitness level of the individual, for example in terms of aerobic or cardiovascular endurance, muscular strength, muscular endurance, flexibility, or body composition. In some embodiments, body shape is given in terms of standardized body types, such as endomorph, mesomorph or ectomorph, for example by so-called somatotyping.

In step 603, the exercise sequence from step 404 (FIG. 4) is processed, based on the exercise category, to determine the performance data, PD. As shown, step 603 may comprise a step 603A, in which the exercise sequence is evaluated in relation to a nominal movement pattern, NMP, for the individual. The NMP may be specific to an exercise and may be obtained, in step 603A, based on the exercise category. The NMP may also be obtained based on the body category, if determined in step 602. The NMP defines parameter values for "ideal" or "acceptable" movements performed by the individual during the exercise. Thus, step 603A may generally be seen to compare the exercise sequence to the NMP. The parameter values may define relative positions and/or orientations between different limbs at different stages during the exercise, durations of different stages of the exercise, etc. Generally, irrespective of the format of the parameter values, the NMP may be seen to define a nominal movement of the 3D poses in the exercise sequence, which corresponds to nominal trajectories of the keypoints 204 (FIG. 2B) in the 3D coordinate system 202 (FIG. 2B). In some embodiments, step 603A comprises detecting and evaluating one or more deviations in time and/or space between the positions of the keypoints in the exercise sequence and the positions of the corresponding keypoints in the nominal trajectories. The respective deviation, if found to be of relevance, may be represented by a performance indicator in the PD.

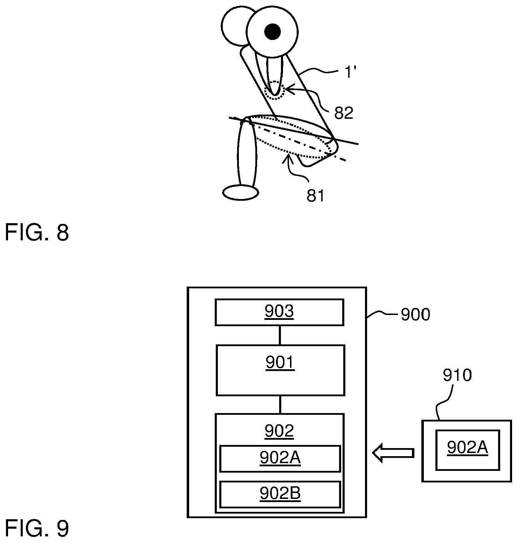

In step 604, which may be part of step 406, the performance indicator(s) determined in step 603 are combined with the animation, which may be done in different ways. In one example, a performance indicator may be presented in plain text. In another example, a performance indicator may be presented as a value of a performance metric calculated in step 603, such as an overall grading of the performance. As shown, step 604 may comprise a step 604A, in which the above-mentioned deviations are indicated visually (highlighted) in the animation to provide corrective feedback, for example by use of contrasting colors or patterns, by encircling deviations, by locally magnifying areas of deviations, etc. The deviation(s) may also be quantified to the user, for example by including ideal 3D poses in the animation of the exercise sequence, and/or by including an ideal trajectory of one or more keypoints in the animation. FIG. 8 shows a momentary 3D pose of the avatar 13' in an animation. Here, two deviations have been identified in the underlying exercise sequence: an incorrect angle of the thigh at the bottom of the squat, and an insufficient stability of the elbow during the squat. The deviations are highlighted in the animation by two performance indicators 81, 82. Indicator 81 represents the ideal angle of the thigh by a dash-dot line in comparison a solid line representing the actual angle. Indicator 82 highlights the instability of the elbow by a dotted marking.

FIG. 7 is a block diagram of an evaluation device 33 in accordance with some embodiments. The evaluation unit 33 comprises a machine learning-based model 33A which has been trained to classify an incoming time series of 3D representations into one or more of a plurality of exercise categories. The model 33A implements step 601 in FIG. 6 and may comprise any known action recognition algorithm. Depending on implementation, the model 33A may output a single exercise category, EC, optionally together with a probability value, P, or a plurality of exercise categories and a probability value of each exercise category. The respective probability value P represents the likelihood that the input data, 3DM, corresponds to the associated exercise category. In FIG. 7, [EC,P] designates one or more exercise categories and associated probability value(s).

The evaluation device 33 further comprises a calculation unit 33B, which is configured to perform step 603 based on 3DM and [EC,P]. As understood from step 603, the calculation unit 33B may be configured to obtain NMP based on EC. If the model 33A provides more than one exercise category, the calculation unit 33B may obtain NMP for the EC with the largest probability value. In the illustrated example, the calculation unit 33B also has access to the body category, BC, of the individual, and is configured to obtain the NMP to match both EC and BC. In accordance with step 603, the calculation unit 33B may process the exercise sequence in relation to NMP to determine the performance data PD.

However, in a simplified embodiment, the calculation unit 33B is configured to determine the PD from the probability value(s) P only. If the largest (or the only) probability value P is above a probability threshold, the calculation unit 33B may generate PD to indicate that the individual's performance is acceptable. Otherwise, PD may be generated to indicate that individual has failed to perform the exercise. The rationale for the simplified embodiment is that the (largest) probability value is generated by the model 33A to reflect how well the exercise sequence matches an exercise category. If the probability is low, it is likely that the exercise is performed with poor form.

The structures and methods disclosed herein may be implemented by hardware or a combination of software and hardware. In some embodiments, such hardware comprises one or more software-controlled computer resources. FIG. 9 schematically depicts such a computer resource 900, which comprises processor circuitry 901, computer memory 902, and a communication interface 903 for input and/or output of data. The communication interface 903 may be configured for wired and/or wireless communication. The processor circuitry 901 may e.g. include one or more of a CPU ("Central Processing Unit"), a DSP ("Digital Signal Processor"), a microprocessor, a microcontroller, an ASIC ("Application-Specific Integrated Circuit"), a combination of discrete analog and/or digital components, or some other programmable logical device, such as an FPGA ("Field Programmable Gate Array"). A control program 901A comprising computer instructions is stored in the memory 902 and executed by the processor circuitry 901 to perform any of the methods, operations, procedures, functions, or steps described in the foregoing. As indicated in FIG. 9, the memory 902 may also store control data 902B for use by the processor circuitry 902, for example a database of NMPs, a database of BCs for different individuals, parameter values for the model 33A, the probability threshold, etc. The control program 902A may be supplied to the computer resource 900 on a computer-readable medium 910, which may be a tangible (non-transitory) product (e.g. magnetic medium, optical disk, read-only memory, flash memory, etc.) or a propagating signal.

While the subject of the present disclosure has been described in connection with what is presently considered to be the most practical embodiments, it is to be understood that the subject of the present disclosure is not to be limited to the disclosed embodiments, but on the contrary, is intended to cover various modifications and equivalent arrangements included within the spirit and the scope of the appended claims.

Further, while operations are depicted in the drawings in a particular order, this should not be understood as requiring that such operations be performed in the particular order shown or in sequential order, or that all illustrated operations be performed, to achieve desirable results. In certain circumstances, parallel processing may be advantageous.

In the following, clauses are recited to summarize some aspects and embodiments as disclosed in the foregoing.

C1. A system for monitoring exercise activity in a gym environment, said system comprising: an image-based monitoring arrangement (10) configured to generate a time series of 3D representations (203) of an individual in the gym environment, wherein each of the 3D representations (203) defines a pose of the individual and comprises positions of a plurality of predefined feature points (204) of the individual in a 3D coordinate system (202); a storage device (31) configured to receive and store at least part of the time series of 3D representations (203), said at least part of the time series corresponding to an exercise performed by the individual, and a replay device (30; 30') configured to retrieve said at least part of the time series from the storage device (31), render an animation of the individual when performing the exercise based on said at least part of the time series, and provide the animation for display.

C2. The system of C1, wherein the replay device (30; 30') is configured to process said at least part of the time series to evaluate performance of the exercise performed by the individual and combine a performance indicator (81, 82) representing the performance with the animation.

C3. The system of C2, wherein the replay device (30; 30') is further configured to process said at least part of the time series to classify the exercise performed by the individual into an exercise category (EC) among a plurality of exercise categories, and to evaluate the performance based on the exercise category (EC).

C4. The system of C3, wherein the replay device (30; 30') is further configured to determine a nominal movement pattern (NMP) for the exercise performed by the individual based on the exercise category (EC), and to evaluate the performance by comparing said at least part of the time series to the nominal movement pattern (NMP).

C5. The system of C4, wherein the nominal movement pattern (NMP) defines nominal trajectories of the positions of the predefined feature points (204), and wherein the replay device (30; 30') is configured to detect and evaluate one or more deviations in time and/or space between the positions of the predefined feature points (204) in said at least part of the time series and the positions of the predefined feature points (204) in the nominal trajectories.

C6. The system of C5, wherein the replay device (30, 30') is configured to combine the performance indicator (81, 82) with the animation to provide a visual indication of the one or more deviations in the animation.

C7. The system of any one of C4-C6, which is further configured to obtain, for the individual, a body category (BC) among a plurality of body categories, and wherein the replay device (30; 30') is configured to determine the nominal movement pattern (NMP) based also on the body category (BC).

C8. The system of any one of C3-C7, wherein the replay device (30; 30') comprises a trained machine learning-based model (33A) which is configured to classify an incoming time series of 3D representations into one or more of the plurality of exercise categories.

C9. The system of C8, wherein the trained machine learning-based model (33A) is configured to generate a probability value (P) for each of the one or more of the plurality of exercise categories, and wherein the replay device (30; 30') is configured to at least partly evaluate the performance based on the probability value (P).

C10. The system of any preceding clause, wherein the replay device (30; 30') is configured to render the animation in response to a display command (DC) entered into the system by a user.

C11. The system of C10, wherein the display command (DC) defines at least one viewing angle onto the individual to be included in the animation and/or a magnification ratio of the individual in the animation.

C12. The system of any preceding clause, wherein the replay device (30; 30') is configured to render the animation as an avatar (1') which is spatially matched to the 3D representations in said at least part of the time series.

C13. The system of C12, which is configured to obtain one or more physiological properties of the individual, wherein the replay device (30; 30') is configured to render the avatar (1') to exhibit the one or more physiological properties.

C14. The system of C13, wherein the one or more physiological properties comprises at least one of weight, height, gender, or body category.

C15. The system of any preceding clause, wherein image-based monitoring arrangement (10) or the storage device (31) is configured to process the time series for detection of a start of the exercise, and wherein the storage device (31) is operable to store an increased number of 3D representations per unit time upon the detection of the start of the exercise.

C16. The system of any preceding clause, wherein the predefined feature points (204) correspond to joints and/or extremities of the individual.

C17. The system of any preceding clause, wherein the system comprises one or more feedback devices (40) operable to display the animation to the individual and/or to a trainer.

C18. The system of any preceding clause, wherein the system comprises two or more feedback devices (40) which are communicatively connected to the replay device (30).

C19. A replay device for use in the system according to any one of C1-C18.

C20. A replay device for use in a system for monitoring exercise activity in a gym environment, wherein the replay device is configured to: retrieve, from a storage device (31), at least part of a time series of 3D representations (203) of an individual in the gym environment, said at least part of the time series corresponding to an exercise performed by the individual, each of the 3D representations (203) defining a pose of the individual and comprising positions of a plurality of predefined feature points (204) of the individual in a 3D coordinate system (202), the time series being generated by an image-based monitoring arrangement (10) in the gym environment; render an animation of the individual when performing the exercise based on said at least part of the time series; and provide the animation for display.

C21. A method of monitoring exercise activity in a gym environment, said method comprising: operating (401) an image-based monitoring arrangement to generate a time series of 3D representations of an individual in the gym environment, wherein each of the 3D representations defines a pose of the individual and comprises positions of a plurality of predefined feature points of the individual in a 3D coordinate system; storing (403) at least part of the time series of 3D representations in a storage device (31), said at least part of the time series corresponding to an exercise performed by the individual; retrieving (404), from the storage device, said at least part of the time series; processing (405, 601) said at least part of the time series to classify the exercise performed by the individual into an exercise category among a plurality of exercise categories; processing (405, 603) said at least part of the time series to evaluate, based on the exercise category, performance of the exercise performed by the individual; rendering (406) an animation of the individual when performing the exercise based on said at least part of the time series and combining (604) a performance indicator representing the performance with the animation; and providing (407) the animation for display.

## Claims

1. A system for monitoring exercise activity in a gym environment, said system comprising:
an image-based monitoring arrangement (10) configured to generate a time series of 3D representations (203) of an individual in the gym environment, wherein each of the 3D representations (203) defines a pose of the individual and comprises positions of a plurality of predefined feature points (204) of the individual in a 3D coordinate system (202),
a storage device (31) configured to receive and store at least part of the time series of 3D representations (203), said at least part of the time series corresponding to an exercise performed by the individual, and
a replay device (30; 30') configured to retrieve said at least part of the time series from the storage device (31), render an animation of the individual when performing the exercise based on said at least part of the time series, and provide the animation for display,
wherein the replay device (30; 30') is further configured to process said at least part of the time series to classify the exercise performed by the individual into an exercise category (EC) among a plurality of exercise categories, process said at least part of the time series to evaluate, based on the exercise category (EC), performance of the exercise performed by the individual, and combine a performance indicator (81, 82) representing the performance with the animation.

2. The system of claim 1, wherein the replay device (30; 30') is further configured to determine a nominal movement pattern (NMP) for the exercise performed by the individual based on the exercise category (EC), and to evaluate the performance by comparing said at least part of the time series to the nominal movement pattern (NMP).

3. The system of claim 2, wherein the nominal movement pattern (NMP) defines nominal trajectories of the positions of the predefined feature points (204), and wherein the replay device (30; 30') is configured to detect and evaluate one or more deviations in time and/or space between the positions of the predefined feature points (204) in said at least part of the time series and the positions of the predefined feature points (204) in the nominal trajectories.

4. The system of claim 3, wherein the replay device (30, 30') is configured to combine the performance indicator (81, 82) with the animation to provide a visual indication of the one or more deviations in the animation.

5. The system of any one of claims 2-4, which is further configured to obtain, for the individual, a body category (BC) among a plurality of body categories, and wherein the replay device (30; 30') is configured to determine the nominal movement pattern (NMP) based also on the body category (BC).

6. The system of any preceding claim, wherein the replay device (30; 30') comprises a trained machine learning-based model (33A) which is configured to classify an incoming time series of 3D representations into one or more of the plurality of exercise categories.

7. The system of claim 6, wherein the trained machine learning-based model (33A) is configured to generate a probability value (P) for each of the one or more of the plurality of exercise categories, and wherein the replay device (30; 30') is configured to at least partly evaluate the performance based on the probability value (P).

8. The system of any preceding claim, wherein the replay device (30; 30') is configured to render the animation in response to a display command (DC) entered into the system by a user.

9. The system of claim 8, wherein the display command (DC) defines at least one viewing angle onto the individual to be included in the animation and/or a magnification ratio of the individual in the animation.

10. The system of any preceding claim, wherein the replay device (30; 30') is configured to render the animation as an avatar (1') which is spatially matched to the 3D representations in said at least part of the time series.

11. The system of claim 10, which is configured to obtain one or more physiological properties of the individual, wherein the replay device (30; 30') is configured to render the avatar (1') to exhibit the one or more physiological properties.

12. The system of any preceding claim, wherein image-based monitoring arrangement (10) or the storage device (31) is configured to process the time series for detection of a start of the exercise, and wherein the storage device (31) is operable to store an increased number of 3D representations per unit time upon the detection of the start of the exercise.

13. The system of any preceding claim, wherein the system comprises two or more feedback devices (40) which are communicatively connected to the replay device (30).

14. A replay device for use in the system according to any one of claims 1-13.

15. A method of monitoring exercise activity in a gym environment, said method comprising:
operating (401) an image-based monitoring arrangement to generate a time series of 3D representations of an individual in the gym environment, wherein each of the 3D representations defines a pose of the individual and comprises positions of a plurality of predefined feature points of the individual in a 3D coordinate system;
storing (403) at least part of the time series of 3D representations in a storage device (31), said at least part of the time series corresponding to an exercise performed by the individual;
retrieving (404), from the storage device, said at least part of the time series;
processing (405, 601) said at least part of the time series to classify the exercise performed by the individual into an exercise category among a plurality of exercise categories;
processing (405, 603) said at least part of the time series to evaluate, based on the exercise category, performance of the exercise performed by the individual;
rendering (406) an animation of the individual when performing the exercise based on said at least part of the time series and combining (604) a performance indicator representing the performance with the animation; and
providing (407) the animation for display.
